# EUROPEAN PATENT SPECIFICATION

(11) **EP 0 421 139 B1**
(45) Date of publication and mention of the grant of the patent: **06.03.1996**
(21) Application number: 90117131.4
(22) Date of filing: 05.09.1990
(51) Int. Cl.: C12N 15/85, C12N 15/70, C12N 9/08, C12N 15/53, G01N 33/53, C12N 1/21, C12N 5/10

(54) **Process for the production of human thyroid peroxidase**
Verfahren zur Herstellung von menschlicher Thiroid-Peroxydase
Procédé pour la production de la péroxydase thyroide humaine

(30) Priority: 05.09.1989 JP 228334/89; 10.05.1990 JP 118770/90
(43) Date of publication of application: 10.04.1991
(60) Divisional application: 94117445.0
(73) Proprietor: NIPPON SUISAN KAISHA, LTD., Tokyo (JP); NISSUI PHARMACEUTICAL CO., LTD., Toshima-ku, Tokyo 170 (JP)
(72) Inventor: Yamashita, Shinya, Fuchu-shi, Toyko (JP); Hata, Junichiro, Koga-shi, Ibaragi-ken (JP); Kabeno, Shouko, Kodaira-shi, Tokyo (JP); Matsumoto, Ken, Sashima-gun, Ibaragi-ken (JP); Yagihashi, Satoru, Souwa-machi, Sashima-gun, Ibaragi-ken (JP); Kato, Hideo, Kasukabe-shi, Saitama-ken (JP)
(74) Representative: Wächtershäuser, Günter, Prof. Dr.

(56) References cited:
- WO-A-91/02061
- EUROPEAN JOURNAL OF CLINICAL INVESTIGATION, vol. 19, no. 2, part II of 2 parts, April 1989, Berlin, DE; P.S. BARNETT et al.: "Cloning and expression of the human thyroid peroxidase gene"
- THE JOURNAL OF CLINICAL ENDOCRINOLOGY & METABOLISM, vol. 62, no. 5, May 1986, pages 928-933, Philadelphia, US; T. KOTANI et al.: "Detection of autoantibodies to thyroid peroxidase in autoimmune thyroid diseases by micro- ELISA and immunoblotting"
- THE EMBO JOURNAL, vol. 6, no. 13, 1987, pages 4193-4196, IRL Press Ltd, Oxford, GB; F. LIBERT et al.: "Tryroperoxidase, an auto-antigen with a mosaic structure made of nuclear and mitochondrial gene modules"
- MOLECULAR ENDOCRINOLOGY, vol. 4, no. 5, May 1990, pages 786-791, Baltimore, US; D. FOTI et al.: "Generation of a biologically active, secreted form of human thyroid peroxidase by site-directed mutagenesis"

## Description

The invention relates to a plasmid suitable for an expression of a membrane bound protein human thyroid peroxidase (hereinafter referred to as hTPO) with a CHO cell, a CHO cell transformed with such a plasmid and a process for the preparation of such a plasmid.

Most of all patients with a thyroid disease has in their serum autoantibodies capable of reacting with thyroglobulin, microsormal antigen and thyrotrophic hormone receptor. Among the autoantibodies, antimicrosormal antibody is quite useful for a diagnosis of autoimmune thyroid diseases, and it has been found that a value of the antibody shows a good interrelation with lymphatic infiltration in thyroid.

Further, it has been reported that the value increases in case of a patient with myasthenia grarsis, lupoid hepatitis, insulin dependent infantile diabetes and the like.

Therefore, a measurement of the antimicrosormal antibody can be utilized for a clinical diagnosis on said diseases.

Hitherto, a measurement of the antimicrosormal antibody has been carried out with various methods and, in general, following methods have been widely accepted, as testing methods.
a) Complement fixation test,
b) Fluorescent antibody test,
c) Passive hemagglutination test,
d) Precipitation test, and
e) E. I. A. (Enzyme Immuno Assay).

Since the nature of microsormal antigen has not sufficiently been elucidated, each of the above conventional methods uses, as an antigen, human thyroid tissue per se or a microsormal fraction obtained by somewhat purifying the tissue, so that it has substantial problems as in following matters.
1) Matter on differences due to origin of thyroid tissue;
2) Matters on humanity due to that the human tissue is employed as the reagent or raw material thereof and on availability of the tissue; and
3) Generation of non-specific reaction due to use of the human tissue or its fraction, which causes instability in sensitivity.

Recently, it has been so estimated that hTPO shall be nature of the antigen to autoantibodies produced in large amount in blood by the patient with thyroid disease. Further, a cloning on cDNA of hTPO has been carried out to make apparent its nucleotide sequence (Number of nucreotides : 3048, Molecular weight : 107000) and a primary structure of amino acids therefor (Shioko KIMURA et al, "Proc. Natl. Acad. Sci. USA", Vol. 84, pages 5555 - 5559, Aug. 1987, Biochemistry).

An object of the invention is to provide a method of measuring antimicrosormal antibodies, which is excellent in reaction specificity to show high sensitivity in measuring.

Another object of the invention is to provide a process for gene engineeringly preparing the hTPO as the nature of microsormal antigen, so that it make possible to supply the same as a reagent in stable and large amount and to finally solve the problems in the conventional methods, due to the humanistic ground and availability.

According to one of aspects of the invention, for attaining the objects, the invention utilizes the hTPO gene cloned by S. KIMURA et al disclosed in said literature, namely the cloned gene is inserted in an expression vector, an animal cell or Escherichia coli is transformed with the vector, and the transformant is cultivated to produce hTPO therein. The measurement of antimicrosormal antibodies can be carried out by using, as antigen, thus produced hTPO to check a reactivity thereof with serum sample.

In the first place, the insertion of expression vector into an animal cell shall be explained. The hTPO gene cloned by S. KIMURA et al has been inserted at EcoRI site of a plasmid (pUC8). In order to produce hTPO in animal cells, an operation is required to provide an initiation and termination of transcription to hTPO. Then , a plasmid (phTPO-1) with the hTPO gene, as disclosed by S. KIMURA et al in said literature, was digested by a restriction enzyme (EcoRI) to obtain a DNA fragment (about 3100bp) of the hTPO gene, While, a plasmid (pSV2-dhfr) known as one of vectors for an expression in animal cells was digested by restriction enzymes (HindIII and BglII) to obtain a DNA fragment (about 4000bp) with a SV40 promoter region and a replication initiation site in Escherichia coli.

In the next place, as a pre-treatment prior to re-construct a plasmid by ligating both of said DNA fragments, it is necessary to make each end of the fragments into a state connectable with each other. This pre-treatment can be carried out in a manner known per se. Namely it can be accomplished by adding a linker at a site cleaved by the restriction enzyme to give a common recognition site, or by treating with a DNA polymerase to make each end in blunt state.

The ligation of the both DNA fragments can be carried out with use of T4 ligase. Escherichia coli (JM109 strain) was cultivated with use of reaction solution to cause a transformation thereof, screened to obtain ampicillin resistance colony, and then recovered a plasmid (pSV2-hTP01) from the transformant. The plasmid is that dhfr (dihydrofolate reductase) gene in the plasmid (pSV2-dhfr) was recombined to hTPO gene, so that the dhfr gene usable as a drug resistance marker should be inserted therein to make complete the plasmid as that for expressing hTPO.

Therefore, the plasmid (pSV2-dhfr) was digested by restriction enzymes (PvuII and BglII) to obtain a DNA fragment (about 1100bp) with a SV40 promoter region and the dhfr gene. While, said re-constructed plasmid (pSV2-hTP01) was cleaved with use of a restriction enzyme (EcoRI).

After pre-treatment in accordance with a manner known per se, both of said DNA fragments were ligated with use of T4 ligase. Escherichia coli (JM109 strain) was transformed with use of the solution for ligation reaction, screened to obtain an ampicillin resistance colony, and recovered a plasmid (pSV2-hTPO-dhfr) from the transformant.

A desired hTPO can be produced in stable and large amount, when the plasmid (pSV2-hTPO-dhfr) is inserted in a CHO cell (DXB11 strain) which is one of eukaryotic cells and widely employed as a host for production of substances with use of gene recombinant engineering techniques, and the resulting transformed cells were cultivated in a manner known per se.

In the following, another case of that an expression plasmid is inserted into Escherichia coli to produce hTPO shall be explained. In this case, also, it is started from the hTPO gene inserted plasmid (phTPO-1) disclosed by S. KIMURA et al, as in aforesaid case, the plasmid is digested and then re-constructed to prepare an expression plasmid. Namely, the plasmid (phTPO-1) is digested with use of restriction enzymes (EcoRI and XhoI) to obtain a DNA fragment (about 2900bp) with a translational region for hTPO gene and 3′-site untranslational region. While, a plasmid (pKK223-3) which is known as an expression vector in Escherichia coli is digested with use of a restriction enzyme (EcoRI) to obtain a DNA fragment.

Prior to a ligation operation for the fragments, following DNA linker (73bp for each strand) is prepared with use of a DNA synthesizer, so as to give a translation initiation codon of ATG which is necessary for an expression of DNA encoding a mature hTPO.

EcoRI
MetArgAlaLeuAlaValLeuSerValThrLeuValMetAlaCysThr
5' -AATTCATGAGAGCGCTCGCTGTGCTGTCTGTCACGCTGGTTATGGCCTGCACA
3' - GTACTCTCGCGAGCGACACGACAGACAGTGCGACCAATACCGGACGTGT
XhoI
GluAlaPhePheProPheIle
GAAGCCTTCTTCCCCTTCATC
CTTCGGAAGAAGGGGAAGTAGAGCT

In the presence of a phosphorylation reaction of the synthetic linker, the fragments as aforesaid are ligated with use of T4 ligase. Further, Escherichia coli (JM109 strain) was transformed with use of the ligating reaction solution, as in aforesaid case and an ampicillin resistance colony was obtained through a screening to recover a plasmid (pNKT-1) from the transformant, as in the manner given in the preceding case.

The desired hTPO can be produced by cultivating said transformed Escherichia coli, or by inserting said recombinant plasmid (pNKT-1) into Escherichia coli (JM109 strain) and cultivating the same.

For expressing in Escherichia coli a polypeptide including a portion for determining the antigen capable to react with autoantibodies of a patient, a part of the hTPO gene and not the entire there of may also be inserted into a plasmid to insert the same, in turn, into Escherichia coli. In this case, a plasmid (pKK233-2) may be employed as the expression vector and this plasmid vector shall be digested by a restriction enzyme (EcoRI) to make the same into a DNA fragment.

Further, if the plasmid (phTPO-1) with hTPO gene, disclosed by S. KIMURA et al in said literature, is digested with use of restriction enzymes (Xhol and AccI), a DNA fragment (about 2450bp) with a part of an open reading frame for the hTPO gene can be obtained, and a DNA fragment (about 970bp) with also a part of the open reading frame for the hTPO gene can be obtained, when the plasmid shall be digested with use of restriction enzymes of SmaI and AccI. Please note that these DNA fragments are obtained through a treatment by the restriction enzyme of AccI, so that those contain no membrane bound region of the hTPO gene.

These DNA fragment can be pre-treated in a manner known per se and ligated with the DNA fragment of said expression vector plasmid to re-construct into recombinant plasmids (pKT-XA and pKT-SA).

When the recombinant plasmid is inserted in Escherichia coli (JM109 strain) and the resulting transformant is cultivated, a substance including at least a part of the hTPO can be produced in the Escherichia coli.

The hTPO produced by the animal cell or Escherichia coli can be isolated and purified in a manner known per se. The resulting hTPO can be employed as the microsormal antigen for the measurement of antimicrosormal antibodies, which utilizes any of conventional methods.

Please note that the hTPO gene is one of membrane bound proteins, so that it expresses in a state bound to cell membrane, when the Escherichia coli or animal cell transformed with the hTPO gene is cultivated. It means that an amount of hTPO production is limited, that a mature cell or Escherichia coli must be broken to obtain hTPO in the form utilizable for the measurement of the antimicrosormal antibodies, to require troublesome purifying operations, even though it may be carried out in a conventional manner, and that there is a fear of that a region with relatively high hydrophobicity, to be considered as a portion binding to membrane may cause a bad influence to a host, when the entire gene of hTPO is inserted in the host of an animal cell or Escherichia coli for expression thereof, although such influence has not been confirmed.
Fig. 1 is an illustration showing manner to construct an expression vector which is suitable to express hTPO protein in an animal cell;
Fig. 2 is patterns showing results of that a molecular weight of hTPO expressed in transformed CHO cells was measured in accordance with western blotting method, in addition to molecular weight of controls, namely hTPO isolated from human thyroid tissue and product of non-transformed CHO cells;
Fig. 3 is an illustration showing a manner to construct an expression vector which is suitable to express in Escherichia coli;
Fig. 4 is an illustration showing manners to conscruct 2 expression vectors, in each of which a part of the hTPO gene is inserted;

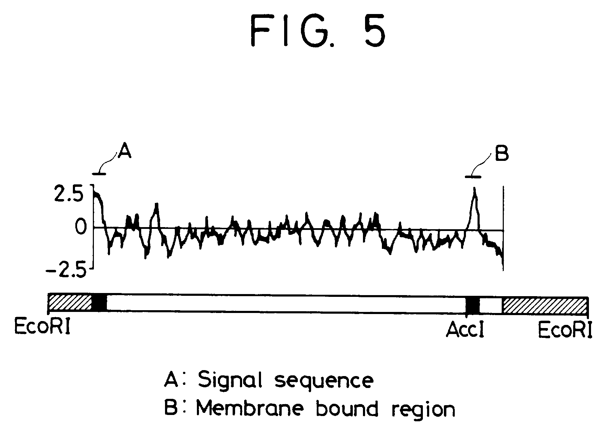

Fig. 5 is a graph showing a result of computical analysis for amino acids encoding hTPO gene, searched from the view point of hydrophilicity - hydrophobicity of the amino acids;

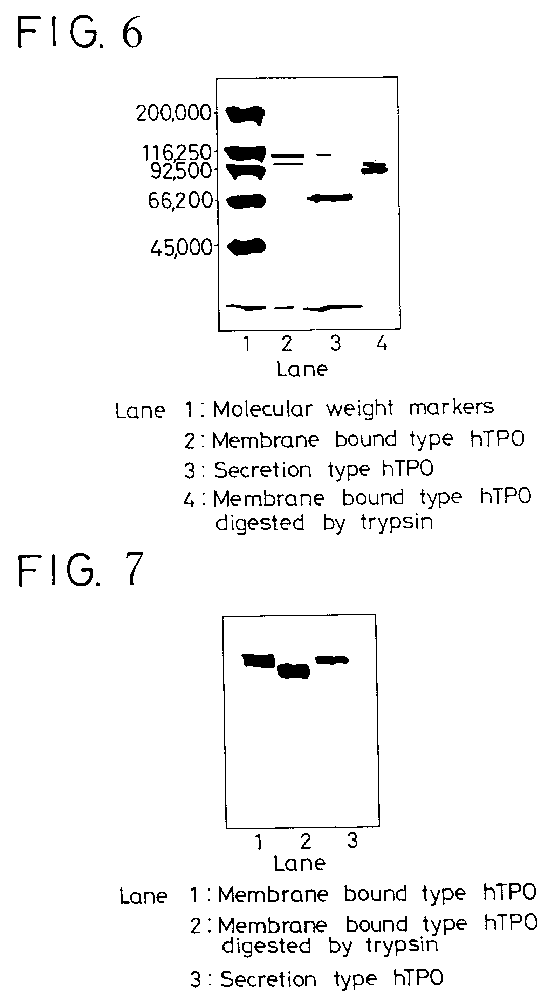

Fig. 6 is patterns showing results of measurements, in accordance with a coomassy dyeing method, on molecular weight of a secretion type hTPO, a natural membrane bound type hTPO, a membrane bound type hTPO digested by trypcin as well as various molecular weight markers;
Fig. 7 is patterns showing results of measurements, in accordance with the western blotting, on molecular weight of the secretion type hTPO, natural membrane bound type hTPO, and membrane bound type hTPO digested by trypcin as well as various molecular weight markers; and

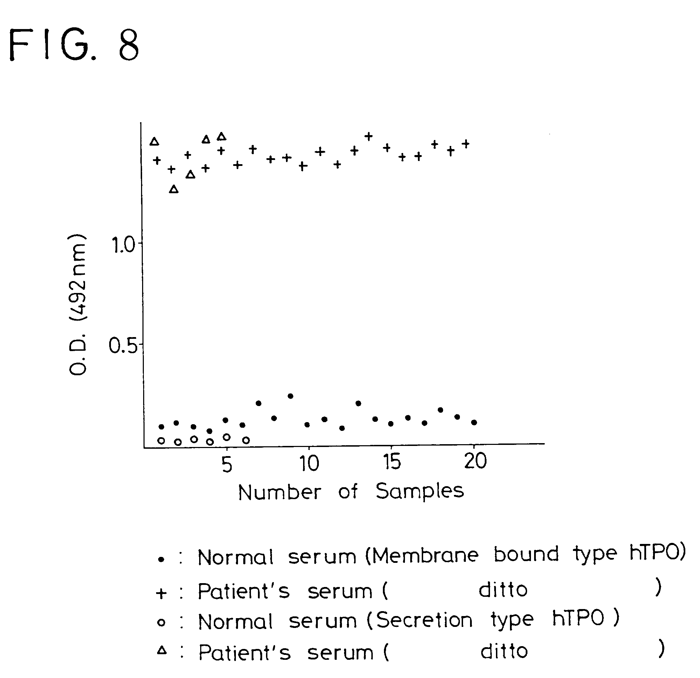

Fig. 8 is a graph showing results of search on reactivity of the secretion type hTPO and natural membrane bound type hTPO with serum samples of normal persons and serum samples of patients with autoimmune thyroid diseases.

The invention will now be further explained by Examples for preparing an hTPO expression vector, for inserting the vector into an animal cell or Escherichia coli, Example for measuring antimicrosormal antibodies, which uses the expressed hTPO, Test Examples and the like. Please note that Examples 1 - 6 as well as Test Examples 1 - 3 relate to a membrane bound type hTPO.

### Example 1

### (Preparation of expression vector)

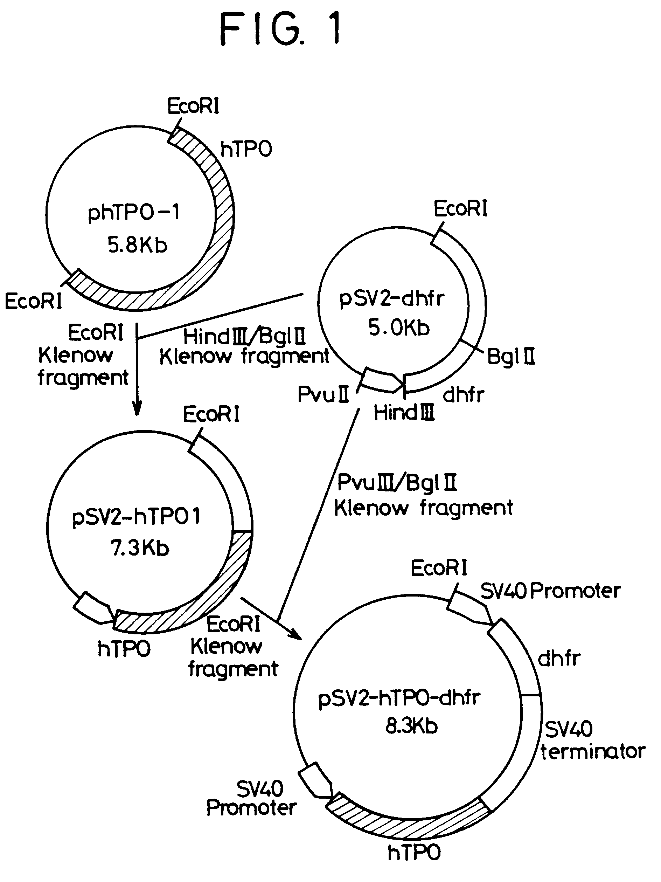

This Example shall be explained with reference to Fig. 1.

An hTPO gene cloned by S. KIMURA et al has been inserted at EcoRI recognition site of a plasmid (phTPO-1) with 5800bp.

To a solution of this plasmid (10µ g) in a mixture (50µ l) of 10mM Toris chloride buffer (pH 7.5), 7mM MgCl₂ and 60mM NaCl, a restriction enzyme (EcoRI, 10 units) was added to digest the plasmid at 37°C for 2 hours. A DNA fragment (about 2µ g) with the hTPO gene and of about 3100bp was obtained from the reaction solution by a DNA separation method of agarose gel electrophoresis.

While, to a solution of a plasmid (pSV2-dhfr, 10µ g) known as one of vectors expressing a products in animal cells, in a mixture (50µ l) of 10mM Toris chloride buffer (pH 7.5), 7mM MgCl₂ and 60mM NaCl, restriction enzymes (HindIII and BglII, each 10 units) were added to digest the plasmid at 37°C for 2 hours. A DNA fragment (about 3µ g) with SV40 promoter region, terminator region and replication origin in Escherichia coli and of about 4000bp was obtained from the reaction solution by the DNA separation method of agarose gel electrophoresis, as in the above.

To each of both DNA fragments (1µ g, respectively), DNA polymerase I (for large fragment) and a mixture of 6.6mM MgCl₂, 6.6mM Toris chloride buffer (pH 7.5), 50mM NaCl, 6.6mM mercaptoethanol and 500µ M dNTP were added to react at 23°C for 60 minutes. After the reaction, an extraction was carried out with phenol to inactivate the enzyme, and purify the DNA with use of the ethanol precipitation method to obtain each DNA fragment (about 0.5µ g) with blunt end.

To a mixture (50µ l) of 70mM Toris chloride buffer (pH 7.5), 10mM MgCl₂, 10mM DTT and 1mM ATP, each of said DNA fragments (0.5 µ g, respectively) was added to dissolve the same, and T4 ligase (5 units) was added therein to react at 16°C for 18 hours. With use of the reaction solution, Escherichia coli (JM109 strain) was transformed and an ampicillin resistance colony was obtained to recover a plasmid from the transformant (This recombinant plasmid had been named as "pSV2-hTPO1").

This plasmid corresponds to that the part of dhfr (dihydrofolate reductase) in the plasmid (pSV2-dhfr) was substituted with the hTPO gene. Since the dhfr gene is useful to give a drug resistance, an operation for inserting this gene into the plasmid (pSV2-hTPO1) shall be explained.

In the first place, the plasmid (pSV2-dhfr, 10µ g) was dissolved in a mixture (50µ l) of 10mM MgCl₂ and 60mM NaCl, and restriction enzymes (PvuII and BglII, each 10 units) were added to digest the plasmid at 37°C. The resulting reaction solution was subjected to agarose gel electrophoresis to obtain a DNA fragment (about 1100bp) with SV40 promoter region and dhfr gene region. While, aforesaid recombinant plasmid (pSV2-hTPO1, 1µ g) was dissolved in a mixture (50µ l) of 10mM Toris chloride buffer (pH 7.5), 7mM MgCl₂ and 60mM NaCl, and a restriction enzyme (EcoRI, 10 units) was added to digest the plasmid at 37°C for 2 hours, so that the plasmid (pSV2-hTPO1) was cleaved at its EcoRI recognition site.

To each of both DNA fragments (1µ g, respectively), DNA polymerase I (for large fragment, 1 unit) and a mixture (50µ l) of 6.6mM MgCl₂, 6.6mM Toris chloride buffer (pH 7.5), 50mM NaCl, 6.6mM mercaptoethanol and 500µ M dNTP were added to react at 23°C for 60 minutes. After the reaction, an extraction was carried out with phenol to inactivate the enzyme, and purify the DNA with use of ethanol precipitation method to obtain each DNA fragment (about 0.5µ g) with blunt end.

To a mixture (50µ l) of 70mM Toris chloride buffer (pH 7.5), 10mm MgCl₂, 10mM DTT and 1mM ATP, each of said DNA fragments (0.5 µ g, respectively) was added to dissolve the same, and T4 ligase (5 units) was added therein to react at 16°C for 18 hours. With use of the reaction solution, Escherichia coli (JM109 strain) was transformed and an ampicillin resistance colony was obtained to recover a plasmid as a desired expression vector, from the transformant (This recombinant plasmid had been named as "pSV2-hTPO-dhfr").

### Reference Example

### (Cultivation of CHO-dhfr cell)

As CHO-dhfr⁻ which is one of eukaryotic cells, DXB11 strain established by Lawrence A. Chasin et al ["Proc. Natl. Acad. Sci. U.S.A.", Vol. 77, No. 7, pages 4126 - 4220 (1980), hereinafter referred to mere as --CHO cell--] was employed.

A medium was prepared by adding NaHCO₃ (2.2g/l) in MEM α medium (marketed by GIBCO Co., No. 410-1900) to prepare a basic medium [hereinafter referred to as --α (+) MEM medium--], and further adding thereto 5% dialyzed bovine fetus serum (hereinafter referred to as --D-FCS--). In this case, the D-FCS was prepared by dialyzing a bovine fetus serum for tissue culturing and marketed by Flow Co. (hereinafter referred to as --FCS--) to a phosphate buffer solution (--PBS-- having a composition of 8.0g/l NaCl, 0.2g/l KCl, 1.15g/l Na₂HPO₄ and 0.2g/l KH₂PO₄). The dialysis was carried out by adding FCS (200ml) to a dialyzing tube (size : 36/32), agitating the PBS (10 litres) kept at 4°C, and changing the PBS in entire amount over 3 times, after lapsed each time period of 10 hours. After completion of the dialysis, the dialized solution was passed through a filter (pore size : 0.2µ m) showing a low protein adsorption to remove possible bacteria. Then, the resulting filtrate was added to the medium.

The cultivation was carried out with use of a CO₂ incuvator, at 37°C, and under atmosphere of 5% CO₂ and saturation with water steam. A passage was carried out with an interval of 3 to 4 days and with a split ratio of 1 : 10 to 1 : 20, by washing with PBS and recover the cells with PBS, in which 0.05% trypcin (marketed by DIFCO Co.) and 0.02% EDTA were added.

### Example 2

### a) Insertion of vector and acquisition of transformants

The CHO cells cultivated and recovered as in Reference Example were washed with 5% D-FCS added α (+) MEM medium and a concentration thereof was adjusted to 5 X 10⁷ cells/ml with use of an insertion buffer [0.25M mannitol, 0.1mM CaCl₂, 0.1mM MgCl₂ and 0.2mM Tris-HCl (pH 7.2)]. While, the vector (PSV2-hTPO-dhfr) obtained in Example 1 was digested by a restriction enzyme (EcoRI), and a digested substance was recovered by ethanol precipitation method to dissolve the same into the insertion buffer to prepare 400µ g/ml solution.

The resulting vector solution and said cell suspension were mixed in equiamount and subjected to an electroporation. The electroporation was carried out with use of a cell fusing device (Type SSH-1) marketed by Shimazu Manufacturing Co., Ltd. and by setting pulse intensity of 3KV/cm, pulse width 50 µ sec, pulse interval 1.0sec and number of pulse 2 times. The mixture (40µ l) was added in an insertion chamber (type C-12) and was left to stand, after the application of the pulse, under room temperature for about 10 minutes and then transferred, in equiamount, to 10 petri dishes (100mm in diameter) which accommodate the 5% D-FCS added α (+) MEM medium. After cultivating for about 48 hours, the medium was changed to α (-) MEM medium (marketed by GIBCO Co. No. 410-2000), in which 5% D-FCS was added, and the cultivation was continued, while changing the medium with a fresh one with an interval of 3 days. After 2 weeks from the initiation of cultivation, a colony of transformed cells was separated with use of a penicillin cup and recovered through trypcin treatment. The colony was subcultured with use of a multidish with 12 cell portions and then subjected to a screening, as stated below.

### b) Screening of hTPO expression cell

The hTPO is a membrane enzyme of human thyroid follicular cells, so that a detection of hTPO expressed on CHO cells was carried out with use of a membrane immuno fluorescent assay (herein after referred to as --MIFA--).

In the first place, the transformed cells cultivated with the multidish were washed with PBS, recovered by a rubber policeman, suspended in PBS (100µ l), transferred to one of cell portions in a titer plate with 96 cell portions, centrifuged (1000rpm for 5 minutes) to remove supernatant therein, and suspended again in PBS (100µ l). After repeating 3 times of the centrifugal and suspending operations, a solution (100µ l) of rabbit anti-hTPO serum diluted 20 volumes with PBS was added and the resulting solution was left to stand for 1 hour at room temperature. After centrifugal washing 3 times with PBS, a solution (50µ l) of FITC labeled anti-rabbit IgG antibody (marketed by Medical and Biological Laboratory Co., Ltd.) diluted 20 volumes with PBS was added and the resulting solution was left to stand for 1 hour at room temperature. The centrifugal washing with PBS was repeated 3 times, and then the cells were suspended in 50µ l of PBS containing 50% glycerol, and a cell accommodating the cell suspension was mounted on a non-fluorescent slide glass to radiate an exciting lightbeam with use of a fluorescent microscope (marketed by Nikon Co., Ltd.).

A fluorescent cell is the desired one transformed and with an ability for producing hTPO.

### Test Example 1

### (Measurement of Molecular weight for produced hTPO)

The hTPO expressed cells confluently propagated in the petri dish [Item (b) in Example 2] were washed with PBS to add an icecooled CSS solution [10ml, containing 10mM Toris chloride buffer (pH 7.5), 150mM NaCl, 0.5% Toriton X-100 and 0.2mM phenylmethylsulphonylfluoride], left to stand for 15 minutes, collected with a rubber policeman, and centrifuged (1000rpm for 10 minutes) to obtain a supernatant as a cell solution.

To the cell solution (30µ l), a sample buffer [15 µ l, containing 60mg/ml SDS, 150mM Toris chloride buffer (pH 6.8) 20% glycerol and 0. 01% BPB] was added, subjected to an electrophoresis, and transcriped to a nitrocellurose membrane. The membrane was dipped in a blocking solution ("Blockace" marketed by Snow Brand Milk Products Co., Ltd.) for one overnight. The membrane was then dipped in a rabbit anti-hTPO serum diluted to 500 fold volumes with T-PBS (0.05% Tween added PBS) and mildly shaked. During the shaking, the solution of T-PBS was changed 3 times with fresh one with an interval of 15 minutes. The membrane was transferred to a solution of T-PBS containing I¹⁵-labeled anti-rabbit IgG antibody (5µ Ci) to further shake mildly for 1 hour.

The resulting membrane was dried by air and an X-ray film was sensitized by the membrane at -70°C to detect a band identifying hTPO.

As controls, CHO cells not inserted the pSV2-hTPO-dhfr vector as well as a natural type hTPO isolated from a human thyroid tissue in accordance with the method as disclosed in J. Clin. Endocrinol. Metab., Vol. 63, page 570 (1986) were employed.

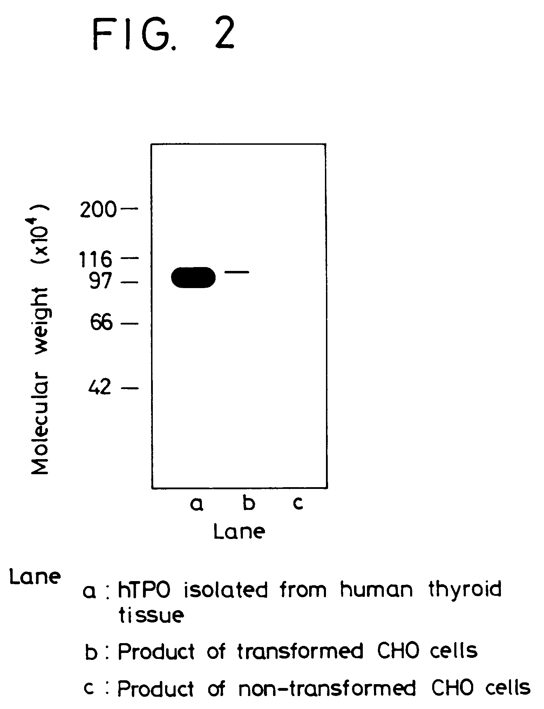

Results are shown in Fig. 2. As apparently from the Figure, it has been found that the molecular weight of hTPO produced by hTPO expression cell disclosed in Example 2 is about 107K daltons and same with that of the natural hTPO and that CHO cells inserted no pSV2-hTPO-dhfr vector does not express hTPO.

### Test Example 2

### (Reactivity of hTPO expressed by hTPO expressing cell with serum sample from patient with autoimmune thyroid disease)

Similar to MIFA described in Item (c) of Example 2 excepting that a serum of a patient with autoimmune thyroid diseases and diluted to 10 fold volumes with PBS was employed in lieu of rabbit anti-hTPO serum and that FITC labeled anti-human IgG antibody was used as a secondary antibody, a fluorescent intensity of cell surface was checked with use of a fluorescent microscope, as an index of an antigenous factor on hTPO produced by the hTPO expression cell. CHO cells inserted no pSV2-hTPO-dhfr were employed as a control.

Results are shown in following Table. As apparently seen therefrom, 24 serum samples among 28 samples show a reactivity to the expressed hTPO.

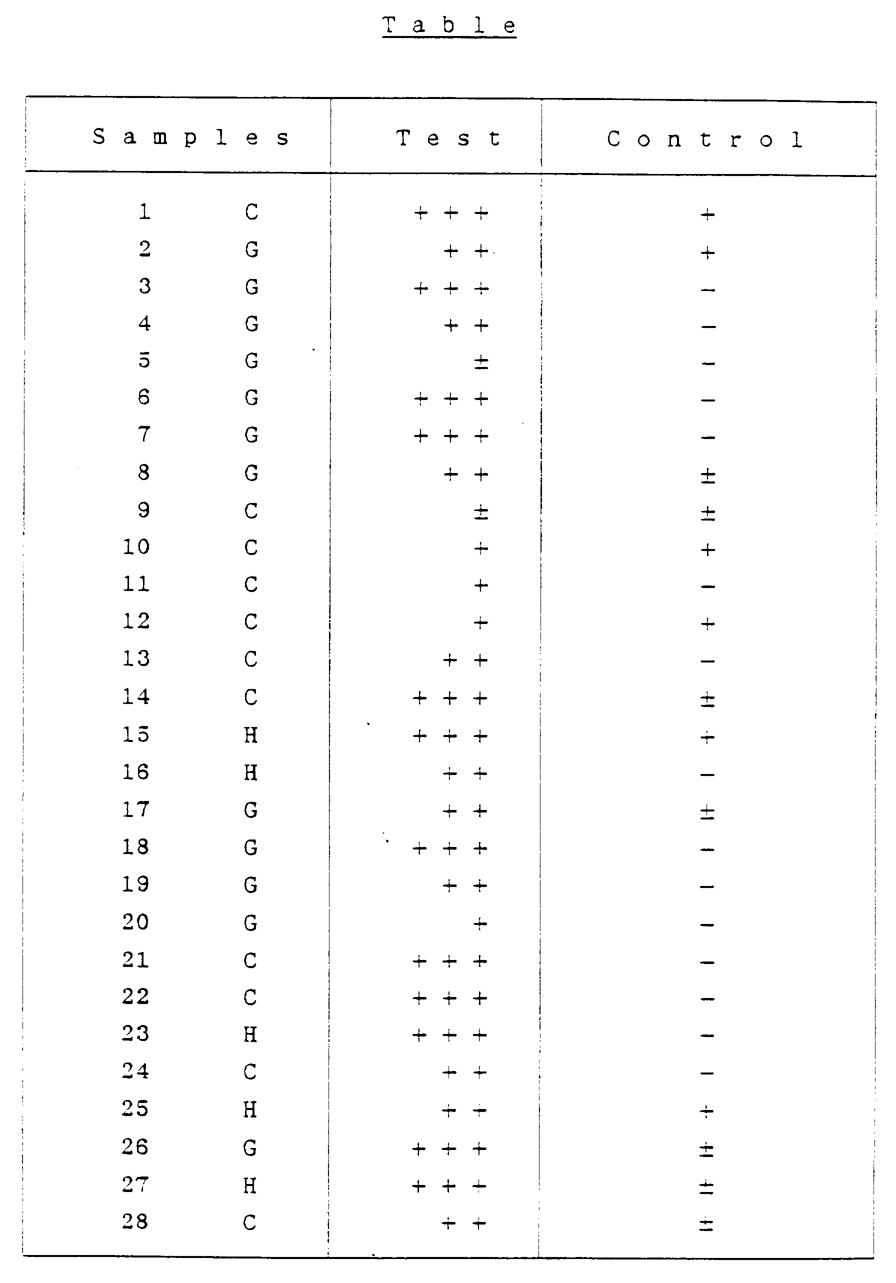

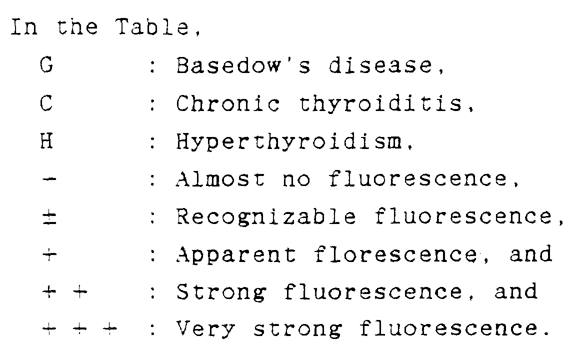

### Example 3

### [Acquisition of cell strain with high hTPO expression ability, from methotrexate (MTX) resistance cell strains]

The hTPO expression cells (1 x 10⁵ cells) obtained by Example 2 was suspended in a medium (8ml) and transferred to a petri dish (10cm in diameter). The medium was prepared by adding 0.05µ M MTX in 5% FCS added α (-) MEM medium. The medium was changed to a fresh one with an interval of 3 days to continue the cultivation for about 2 weeks. A formed methotrexate resistance colony was recovered with a penicillin cup method to screen a cell strain showing high hTPO expression ability. In this case, some cell strains show a fluorescent intensity higher than that obtained by Example 2, so that such cell strains were recovered as those with high hTPO expression ability. Among 24 cell strains expressing hTPO, 4 strains show a high hTPO expression ability.

### Example 4

### (Preparation of expression vector)

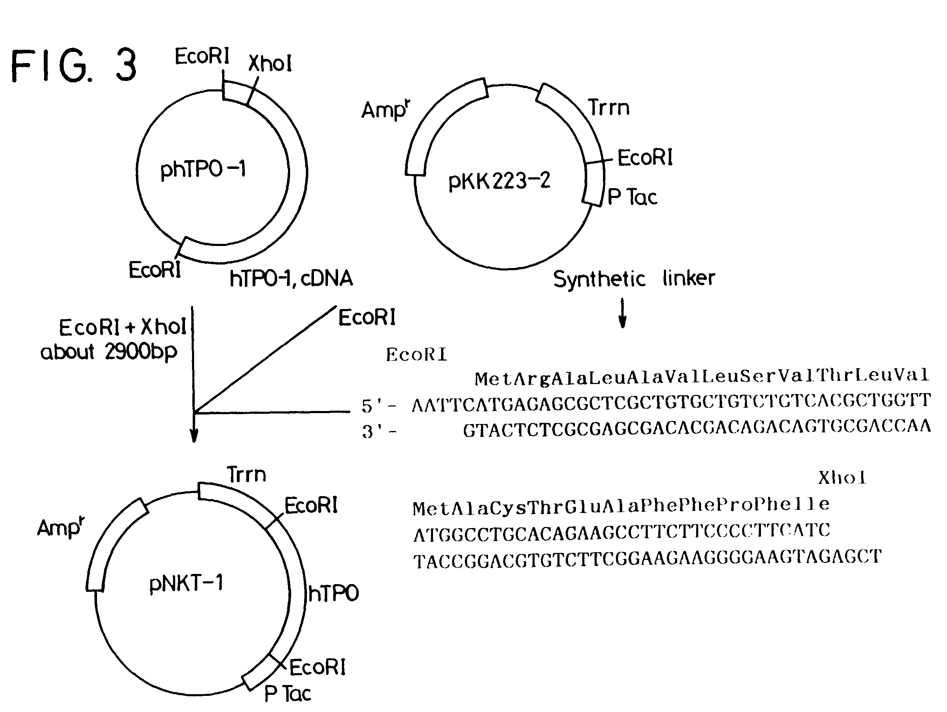

This Example shall be explained with reference to Fig. 3.

In the first place, a hTPO gene inserted plasmid (phTPO-1, 5 µ g) disclosed by S. KIMURA et al was dissolved in a mixture (50 µ l) of 10mM Toris chloride buffer (pH 7.5), 7mM MgCl₂ and 100mM NaCl, and then restriction enzymes (EcoRI and XhoI, each 10 units) were added to the solution to digest the plasmid at 37°C for 2 hours. From the reaction solution, a DNA fragment (1µ g, about 2900bp) with hTPO translational region and 3'-site non-translational region was obtained by a DNA separation method of agarose gel electrophoresis.

While, to a solution of a plasmid (pKK223-3, 1µ g, marketed by Pharmacia Co.), known as one of vectors expressing a product In Escherichia coli, in a mixture (50µ l) of 10mM Toris chloride buffer (pH 7.5), 7mM MgCl₂ and 100mM NaCl, a restriction enzyme (EcoRI, 10 units) was added to digest the plasmid at 37°C for 2 hours. After inactivation of the enzyme by phenol extraction, ethanol precipitation method was carried out to purify and obtain a DNA fragment (pKK223-2, about 0.5µ g).

Following DNA linker (73bp for each strand) was prepared with use of a DNA synthesizer, so as to give a translational initiation codon of ATG which is necessary for expressing a DNA encoding a mature hTPO and as a pre-treatment for causing a ligation of said DNA fragments.

EcoRI
MetArgAlaLeuAlaValLeuSerValThrLeuValMetAlaCysThr
5' -AATTCATGAGAGCGCTCGCTGTGCTGTCTGTCACGCTGGTTATGGCCTGCACA
3' -     GTACTCTCGCGAGCGACACGACAGACAGTGCGACCAATACCGGACGTGT
XhoI
GluAlaPhePheProPheIle
GAAGCCTTCTTCCCCTTCATC
CTTCGGAAGAAGGGGAAGTAGAGCT

The synthesis was carried out by preparing each single-stranded DNA of 73 nucleotides, with use of the DNA synthesizer marketed by ABI Co., dissolving each strand (50pmol) in a mixture (50µ l) of 50mM Toris chloride buffer (pH 7.5), 10mM MgCl₂, 10mM DTT and 1mM ATP, adding T4 nucleotide kinase (5 units, marketed by Takara Shuzo Co., Ltd.) to phosphorylate at 37°C for 1 hour.

For a ligation of aforesaid two DNA fragments (0.5µ g, respectively) obtained by cleaving the concerned plasmid, each of the DNA fragments was taken by 0.5µ g, dissolved in a mixture (50 µ l) of 70mm Toris chloride buffer (pH 7.5), 10mm MgCl₂, 10mM DTT and 1mM ATP, and then the phosphorylation reaction solution of said synthetic linker (each 1µ l) was added thereto. To the resulting mixture, T4 ligase (5 units) was added to cause ligation reaction at 16°C for 18 hours. Escherichia coli (JM109 strain) was transformed with use of the resulting reaction solution and an ampicillin resistance colony was obtained through a screening to recover a plasmid (pNKT-1) from recovered transformant. A structure of this plasmid was checked through digestive experiments using various restriction enzymes.

### Example 5

### [Escherichia coli with plasmid (pNKT-1) and expression of hTPO by Escherichia coli]

The transformant described in Example 4 is the hTPO expression Escherichia coli. A desired hTPO expression Escherichia coli can also be prepared by inserting the recombinant plasmid (pNKT-1) obtained by Example 4 into Escherichia coli, in a manner known per se.

The Escherichia coli with the the plasmid (pNKT-1) was planted in LB medium (10g/l tryptone, 5g/l yeast extract and 5g/l NaCl), cultivated at 37°C to add isopropylthiogalactopyranoside (IPTG) in concentration of 50µ g/ml at a middle of logarithmic growth phase thereof and to continue the cultivation at 37°C, and then centrifuged to collect a solid. The solid was suspended in a sample buffer of laemli, subjected to SDS polyacryloamide gel electrophoresis, subjected to western blotting in accordance with the manner as described in "Proc. Natl. Acad. Sci. USA" Vol. 76, page 4350 (1979), and subjected to peroxidase dyeing in accordance with the manner as described by TABE in the literature "Saibou Kougaku" (translated as --Cell Engineerings--), Vol. 2, page 1061 (1983) to detect a band at position of molecular weight of 107K daltons. This means that the Escherichia coli with plasmid (pNKT-1) expresses hTPO.

### Example 6

### (Expression of polypeptide constituting a part of hTPO)

An experiment was carried out for the purpose of expressing in Escherichia coli a polypeptide containing an antigen determination site reacting with an autoantibody of patient, and for producing not the entire DNA for hTPO but a part thereof.

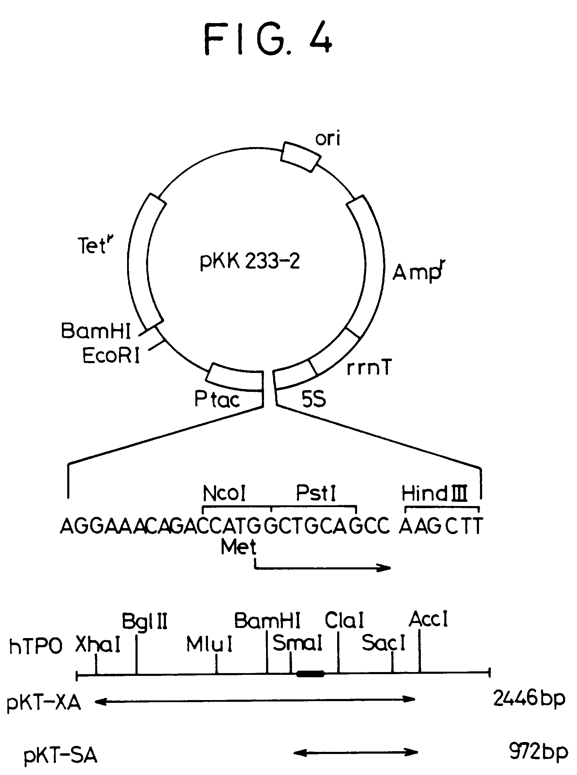

This Example shall be explained with reference to Fig. 4. In the first place, a hTPO gene inserted plasmid (phTPO-1, 5 µ g) disclosed by S. KIMURA et al was dissolved in a mixture (50 µ l) of 10mm Toris chloride buffer (pH 7.5), 7mM MgCl₂ and 100mM NaCl, and then restriction enzymes (XhoI and AccI, each 10 units) were added to the solution to digest the plasmid at 37°C for 2 hours. From the reaction solution, a DNA fragment (about 1µ g, 2446bp) with a part of hTPO translational region was obtained by a DNA separation method of agarose gel electrophoresis.

Similar to the above using the plasmid (phTPO-1, 5µ g) but using restriction enzymes of SmaI and AccI (each 10 units), a DNA fragment (about 1µ g, 972bp) with a part of hTPO translational region was obtained from a reaction solution.

z Each of the both DNA fragments was dissolved in a mixture (50µ l) of 6.6mM MgCl₂, 6,6mM Toris chloride buffer (pH 7.5), 50mM NaCl, 6.6mM mercaptoethanol and 500µ M dNTP, and DNA polymerase I (for large fragment, 1 unit) was added to the solution to react at 23°C for 60 minutes. A phenol extraction was carried out on the reaction solution to inactivate the enzyme and the resulting extract was purified in accordance with ethanol precipitation method to obtain a DNA fragment (each 0.5µ g) with a blunt end.

While, to a solution of a plasmid (pKK223-2, 1µ g, marketed by Pharmacia Co.) in a mixture (50µ l) of 10mm Toris chloride buffer (pH 7.5), 7mM MgCl₂ and 100mM NaCl, a restriction enzyme (NcoI, 10 units) was added to digest the plasmid at 37°C for 2 hours. After inactivation of the enzyme by phenol extraction, ethanol precipitation method was carried out to purify and obtain a DNA fragment (pKK223-2, about 0.5µ g). The DNA fragment (about 1µ g) was dissolved in a mixture (50µ l) of 6.6mM MgCl₂, 6,6mM Toris chloride buffer (pH 7.5), 50mM NaCl, 6.6mM mercaptoethanol and 500µ M dNTP, and DNA polymerase I (1 unit) was added to the solution. A phenol extraction was carried out on the reaction solution to inactivate the enzyme and the resulting extract was purified in accordance with ethanol precipitation method to obtain a DNA fragment (about 0.5µ g) with a blunt end (this DNA fragment shall be referred to as "vector DNA fragment" for the sake of explanation).

The vector DNA is ligated with one of aforesaid 2 DNA fragments (with 2446bp and 972bp, respectively and with a blunt end) to re-construct 2 different expression vectors, but a manner of the ligation is same with each other. Namely, the vector DNA fragment and one of the DNA fragments, for instance that with 2446bp are dissolved in a mixture (50µ l) of 70mM Toris chloride buffer (pH 7.5), 10mM MgCl₂, 10mM DTT and 1mM ATP, and T4 ligase (5 units) was added thereto to react at 16°C for 18 hours to cause a ligation of the fragments. Escherichia coli (JM109 strain) was transformed with use of the resulting reaction solution and an ampicillin resistance colony was obtained through a screening. This transformant is the desired transformed Escherichia coli.

When a plasmid is recovered from this transformant, a desired recombinant expression plasmid (pKT-XA) can be obtained. While, if starting from the DNA fragment with 972bp, another desired plasmid (pKT-SA) can finally be obtained.

A structure of the plasmid (pKT-XA and pKT-SA) has been checked and confirmed through a digestion with use of various restriction enzymes.

### Test Example 3

### (Production of polypeptide containing a part of hTPO)

The transformant (Escherichia coli with plasmid (pKT-XA or pKT-SA) obtained by Example 6 was planted in LB medium, cultivated at 37°C to add IPTG in concentration of 50µ g/ml at a middle of logarithmic growth phase thereof and to continue the cultivation at 37°C , and then centrifuged to collect a solid. Similar to the manner as described in Example 5, the solid was suspended in a sample buffer of laemli, subjected to SDS polyacryloamide gel electrophoresis, subjected to western blotting using anti-hTPO polyclonal antibody, and subjected to peroxidase dyeing, so that bands at positions of molecular weight of 90K daltons and 49K daltons were detected for pKT-XA and pKT-SA, respectively. These bands can not be detected on a control of Escherichia coli with a plasmid of neither pKT-XA nor pKT-SA.

From the above, it has been confirmed that the Escherichia coli with the plasmid of pKT-XA or pKT-SA produces a part of polypeptide encoding hTPO.

The deposition numbers on the CHO-cell-lines have been given, as follows:
a) CHO cell-line transformed with a vector plasmid to express a membrane bound type hTPO:
   Deposition No. FERN BP-3076
   Deposition date: August 24, 1990
b) CHO cell-line transformed with a vector plasmid to express a secretion type hTPO:
   Deposition No. FERM BP-3077
   Deposition date: August 24, 1990

## Claims

1. A plasmid suitable for an expression of a membrane bound protein human thyroid peroxidase with a CHO cell, which comprises:
(a) a DNA sequence coding for a first protein human thyroid peroxidase;
(b) a first promoter sequence controlling an expression of the DNA sequence coding for the first protein;
(c) a DNA sequence coding for a second protein dihydrofolate reductase;
(d) a second promoter sequence controlling an expression of the DNA sequence coding for the second protein, said DNA sequence coding for the first protein and said DNA sequence coding for the second protein being situated between said first promoter sequence and said second promoter sequence, and said both promoter sequences facing to the opposite direction each other; and
(e) the only one terminator sequence common to said DNA sequence coding for the first protein and said DNA sequence coding for the second protein, being situated between said both sequences coding for each protein.

2. The plasmid according to Claim 1, wherein said first and second promoter sequences are each derived from an SV40 promoter sequence, and said terminator sequence is derived from an SV40 terminator sequence.

3. The plasmid pSV2-hTPO-dhfr as shown in Fig. 1 of about 8.3kb suitable for an expression of a membrane bound protein human thyroid peroxidase with a CHO cell.

4. A CHO cell transformed with the plasmid according to Claim 1.

5. A CHO cell transformed with the plasmid according to Claim 3.

6. A process for preparation of a plasmid suitable for an expression of membrane bound protein human thyroid peroxidase in a CHO cell, which comprises the steps of:
(a) cleaving plasmid phTPO-1 with restriction enzyme EcoRI to obtain a DNA fragment coding for human thyroid peroxidase;
(b) cleaving plasmid pSV2-dhfr with restriction enzymes HindIII and BglII to obtain a DNA fragment which comprises an SV40 promoter sequence, terminator sequence and replication origin sequence in E. coli;
(c) respectively reacting each DNA fragment obtained by said step(a) and step(b) with the Klenow fragment of E. coli in the presence of DNA polymerase I to produce blunt ends on each of the DNA fragments;
(d) ligating with T4 ligase resulting two kinds of DNA fragments obtained by said step(c) to obtain reactant solution;
(e) transforming E. coli with the reactant solution obtained by said step(d) to obtain an ampicillin resistant transformant;
(f) recovering plasmid pSV2-hTPO1 from the transformant obtained by said step(e);
(g) cleaving another plasmid pSV2-dhfr with restriction enzymes PvuII and BglII to obtain a DNA fragment comprising an SV40 promoter sequence and a dihydrofolate reductase sequence;
(h) cleaving the plasmid pSV2-hTPO1 obtained by said step(f) with restriction enzyme EcoRI to obtain a DNA fragment comprising sequence coding for human thyroid peroxidase;
(i) respectively reacting each DNA fragment obtained by said step(g) and obtained by said step(h) with the Klenow fragment of E. coli in the presence of DNA polymerase I to produce blunt ends on each of the DNA fragments;
(j) ligating with T4 ligase resulting two kinds of DNA fragments obtained by said step(i) to obtain reactant solution;
(k) transforming E. coli with the reactant solution obtained by said step(j) to obtain an ampicillin resistant transformant; and
(1) recovering plasmid pSV2-hTPO-dhfr of 8.3kb from the transformant obtained by said step(k).

## Patentansprüche

1. Plasmid, das in einer CHO-Zelle zur Expression eines membrangebundenen, menschlichen Thyroidperoxidase-Proteins geeignet ist, und umfaßt:
(a) eine DNA-Sequenz, die als erstes Protein menschliche Thyroidperoxidase kodiert;
(b) eine erste Promotorsequenz, die eine Expression der DNA-Sequenz, die das erste Protein kodiert, steuert;
(c) eine DNA-Sequenz, die als zweites Protein Dihydrofolatreduktase kodiert;
(d) eine zweite Promotorsequenz, die eine Expression der DNA-Sequenz, die das zweite Protein kodiert, steuert, wobei die DNA-Sequenz, die das erste Protein kodiert, und die DNA-Sequenz, die das zweite Protein kodiert, zwischen der ersten Promotorsequenz und der zweiten Promotersequenz angeordnet sind, und beide Promotorsequenzen in entgegengesetzter Richtung zueinander ausgerichtet sind; und
(e) die einzige gemeinsame Terminatorsequenz der DNA-Sequenz, die das erste Protein kodiert und der DNA-Sequenz, die das zweite Protein kodiert, zwischen beiden Sequenzen angeordnet ist, die die jeweiligen Proteine kodieren.

2. Plasmid gemäß Anspruch 1, wobei die erste und zweite Promotorsequenz jeweils von einer SV40-Promotorsequenz abgeleitet ist und die Terminatorsequenz von einer SV40-Terminatorsequenz abgeleitet ist.

3. Plasmid pSV2-hTPO-dhfr, wie in Fig. 1 gezeigt, mit ungefähr 8,3 kb, das in einer CHO-Zelle zur Expression eines membrangebundenen, menschlichen Thyroidperoxidase-Proteins geeignet ist.

4. CHO-Zelle, die mit dem Plasmid gemäß Anspruch 1 transformiert ist.

5. CHO-Zelle, die mit dem Plasmid gemäß Anspruch 3 transformiert ist.

6. Verfahren zur Herstellung eines Plasmids, das in einer CHO-Zelle zur Expression von membrangebundenem, menschlichen Thyroidperoxidase-Protein geeignet ist und folgende Schritte umfaßt:
(a) Spaltung eines Plasmids phTPO-1 mit einem Restriktionsenzym EcoRI, um ein DNA-Fragment, das eine menschliche Thyroidperoxidase kodiert, zu erhalten;
(b) Spaltung des Plasmids pSV2-dhfr mit Restriktionsenzymen HindIII und BglII, um ein DNA-Fragment zu erhalten, das eine SV40-Promotorsequenz, eine Terminatorsequenz und eine Replikationsursprungssequenz in E. coli umfaßt;
(c) Umsetzung eines jeden DNA-Fragments, das durch die Schritte (a) und (b) erhalten wurde, jeweils mit dem Klenow-Fragment von E. coli in Gegenwart von DNA-Polymerase I, um stumpfe Enden an den DNA-Fragmenten herzustellen;
(d) Ligation mit T4-Ligase von zwei Arten von DNA-Fragmenten, die durch Schritt (c) erhalten wurden, um eine Reaktionslösung zu erhalten;
(e) Transformation von E. coli mit der Reaktionslösung, die durch Schritt (d) erhalten wurde, um eine Ampicillinresistente Transformante zu erhalten;
(f) Gewinnung eines Plasmids pSV2-hTPO-1 aus der Transformante, die in Schritt (e) erhalten wurde;
(g) Spaltung eines weiteren Plasmids pSV2-dhfr mit Restriktionsenzymen PvuII und BglII, um ein DNA-Fragment zu erhalten, das eine SV40-Promotorsequenz und eine Dihydrofolatreduktasesequenz enthält;
(h) Spaltung des Plasmids pSV2-hTPO1, das in Schritt (f) erhalten wurde mit dem Restriktionsenzym EcoRI, um ein DNA-Fragment zu erhalten, das eine Sequenz umfaßt, die eine menschliche Thyroidperoxidase kodiert;
(i) Umsetzung eines jeden DNA-Fragments, das in Schritt (g) und (h) erhalten wurde mit dem Klenow-Fragment von E. coli, in Gegenwart von DNA-Polymerase I, um stumpfe Enden an den DNA-Fragmenten zu erhalten;
(j) Ligation mit T4-Ligase von zwei Arten von DNA-Fragmenten, die in Schritt (i) erhalten wurden, um eine Reaktionslösung zu erhalten;
(k) Transformation von E. coli mit der Reaktionslösung, die in Schritt (j) erhalten wurde, um eine Ampicillin-resistente Transformante zu erhalten; und
(1) Gewinnung eines Plasmids pSV2-hTPO-dhfr mit 8,3 kb aus der Transformante, die in Schritt (k) erhalten wurde.

## Revendications

1. Plasmide approprié pour une expression d'une protéine liée à une membrane, la peroxydase thyroïdienne humaine, avec une cellule CHO, qui comprend :
(a) une séquence d'ADN codant pour une première protéine peroxydase thyroïdienne humaine ;
(b) une première séquence promoteur contrôlant une expression de la séquence d'ADN codant pour la première protéine ;
(c) une séquence d'ADN codant pour une seconde protéine dihydrofolate réductase ;
(d) une seconde séquence promoteur contrôlant une expression de la séquence d'ADN codant pour la seconde protéine, ladite séquence d'ADN codant pour la première protéine et ladite séquence d'ADN codant pour la seconde protéine étant situées entre ladite première séquence promoteur et ladite seconde séquence promoteur, et lesdites deux séquences promoteurs étant tournées dans des directions opposées l'une à l'autre ; et
(e) la seule et unique séquence terminateur commune à ladite séquence d'ADN codant pour la première protéine et ladite séquence d'ADN codant pour la seconde protéine étant située entre lesdites deux séquences codant pour chaque protéine.

2. Plasmide selon la revendication 1, dans lequel lesdites première et seconde séquences promoteurs sont chacunes issues d'une séquence promoteur de SV40, et ladite séquence terminateur est issue d'une séquence terminateur de SV40.

3. Plasmide pSV2-hTPO-dhfr tel que représenté sur la Figure 1, d'environ 8,3 kb, approprié pour une expression d'une protéine liée à une membrane, la peroxydase thyroïdienne humaine, avec une cellule CHO.

4. Cellule CHO transformée par le plasmide tel que défini à la revendication 1.

5. Cellule CHO transformée par le plasmide tel que défini à la revendication 3.

6. Procédé de préparation d'un plasmide approprié pour une expression d'une protéine liée à une membrane, la peroxydase thyroïdienne humaine dans une cellule CHO, qui comprend les étapes consistant à :
(a) cliver le plasmide phTPO-1 par l'enzyme de restriction EcoRI, pour obtenir un fragment d'ADN codant pour la peroxydase thyroïdienne humaine ;
(b) cliver le plasmide pSV2-dhfr par les enzymes de restriction HindIII et BglII, pour obtenir un fragment d'ADN qui comprend une séquence promoteur, une séquence terminateur et une séquence d'origine de réplication de SV40 dans E. coli ;
(c) faire réagir respectivement chaque fragment d'ADN obtenu par ladite étape (a) et ladite étape (b) avec le fragment de Klenow d'E. coli, en présence d'ADN polymérase I, pour produire des extrémités droites sur chacun des fragments d'ADN ;
(d) souder avec de la ligase T4 les deux sortes résultantes de fragments d'ADN obtenus par ladite étape (c), pour obtenir une solution de réactifs ;
(e) transformer E. coli avec la solution de réactifs obtenue par ladite étape (d), pour obtenir un transformant résistant à l'ampicilline ;
(f) récupérer le plasmide psv2-hTPO1 à partir du transformant obtenu par ladite étape (e) ;
(g) cliver un autre plasmide pSV2-dhfr par les enzymes de restriction PvuII et BglII, pour obtenir un fragment d'ADN comprenant une séquence promoteur de SV40 et une séquence de dihydrofolate réductase ;
(h) cliver le plasmide pSV2-hTPO1 obtenu par ladite étape (f) par l'enzyme de restriction EcoRI, pour obtenir un fragment d'ADN comprenant une séquence codant pour la peroxydase thyroïdienne humaine ;
(i) faire réagir respectivement chaque fragment d'ADN obtenu par ladite étape (g) et obtenu par ladite étape (h) avec le fragment de Klenow d'E. coli en présence d'ADN polymérase I, pour obtenir des extrémités droites sur chacun des fragments d'ADN ;
(j) souder avec la ligase T4 les deux sortes résultantes de fragments d'ADN obtenus par ladite étape (i), pour obtenir une solution de réactifs ;
(k) transformer E. coli par la solution de réactifs obtenue par ladite étape (j), pour obtenir un transformant résistant à l'ampicilline ; et
(1) récupérer le plasmide pSV2-hTPO-dhfr de 8,3 kb à partir du transformant obtenu par ladite étape (k).
